(19)

**Europäisches Patentamt**
**European Patent Office**
**Office européen des brevets**

(11) **EP 4 400 037 B1**

(12) **EUROPEAN PATENT SPECIFICATION**

(45) Date of publication and mention
of the grant of the patent:
**11.03.2026  Bulletin 2026/11**

(21) Application number: **23220511.2**

(22) Date of filing: **28.12.2023**

(51) International Patent Classification (IPC):
*A61B 5/021* *(2006.01)*        *A61B 5/022* *(2006.01)*

(52) Cooperative Patent Classification (CPC):
**A61B 5/02225; A61B 5/02141; A61B 5/0225;**
A61B 2560/0209

(54) **ELECTRONIC SPHYGMOMANOMETER WITH INTEGRATED AIR PUMP**

ELEKTRONISCHES SPHYGMOMANOMETER MIT INTEGRIERTER LUFTPUMPE

SPHYGMOMANOMÈTRE ÉLECTRONIQUE À POMPE À AIR INTÉGRÉE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **16.01.2023   CN 202310058853**

(43) Date of publication of application:
**17.07.2024   Bulletin 2024/29**

(73) Proprietor: **Yasee Biomedical Co., Ltd.
Qingdao City, Shandong 266112 (CN)**

(72) Inventor:
• **The inventors have waived their right to be thus
mentioned.**

(74) Representative: **Eder Schieschke & Partner mbB
Patentanwälte
Elisabethstraße 34
80796 München (DE)**

(56) References cited:
WO-A1-2021/208745      CN-A- 106 983 503
CN-U- 209 611 120      US-A1- 2018 098 741

**Description**

**BACKGROUND OF THE APPLICATION**

**1. Technical Field**

The present disclosure generally relates to medical instruments, and more particularly to an electronic sphygmomanometer with integrated air pump and a control method thereof.

**2. Description of Related Art**

Cardiovascular disease is responsible for a high fatality rate globally. Hypertension, as a typical section of cardiovascular disease, has been recognized as the most critical illness second only to diabetes. Pulse wave signals are signals varying with regularly variations of arteries and cardiac contraction. Currently, researchers of cardiovascular disease usually monitoring human blood pressure and assessing elasticity of arteries by recording pulse waveforms and extracting features of the waveforms.

Existing electronic sphygmomanometers are generally based on the oscillometric method and implement step-down measurement, which involves inflating the sleeve with the air pump for pressurization to block the blood flow in the brachial artery, and then slowly deflating the sleeve for depressurization while collecting pressure data. Since slow depressurization during step-down measurement has to be performed with the deflation speed precisely controlled, a solenoid valve and a quick exhaust valve are always provided for controlled deflation. Consequently, the master controller in such an electronic sphygmomanometer has to control many components during measurement, which means increased computing loads, and forms a common problem among existing step-down electronic sphygmomanometers.

With development of the IoT technology, wireless communication has increasingly been recognized as an essential function for electronic sphygmomanometers. For example, CN111012323A discloses an apparatus for estimating blood pressure and an apparatus for supporting blood pressure estimation. In the known apparatuses, the communication interface is configured to communicate with external devices through a wired/wireless communication means under the control of the processor for transmitting and receiving various data. The communication means may include Bluetooth, Bluetooth Low Energy (BLE), near-field communication (NFC), wireless local area networks (WLAN), etc. However, none of these communication solutions can be achieved without hardware support, namely a corresponding chip. This reflects the popular hardware configuration of electronic sphygmomanometers capable of wireless communication. To be specific, an MCU control chip is used as the controller/processor and communication-related tasks are performed by corresponding communication-related chips. However, some chips related to communication, particularly Bluetooth chips, have computing power sufficient to cover at least a part of data-processing and analysis tasks. This means that the Bluetooth chips currently provided on electronic sphygmomanometers are not fully developed and used in terms of function, leading to functional overflow of MCU chips in these sphygmomanometers. The unreasonable resource allocation in existing electronic sphygmomanometer supporting wireless transmission, and in particular Bluetooth transmission, accounts for considerable wasted costs.

Besides, most existing electronic sphygmomanometers have their display screen arranged on the surface of its main body. The presence of a display screen not only increases the surface area of the sphygmomanometer but also adds loads to the battery.

As an improvement to the foregoing problem, some existing sphygmomanometers have removed the display unit from the main body. For example, the blood pressure measuring apparatus with a wireless transmission of pressure signals disclosed in US20060111639A1 comprises a wireless communication module adopting the standard communication protocol so that it can have two-way data connection with "another electronic product" that is equipped with a display device for displaying blood-pressure measurement values. However, the known apparatus has its signal amplifier followed by an AD converter. The AD converter, the main controller, and the wireless communication module chip unavoidably cause high standby power consumption, and can lead to the inconvenience that the apparatus has to operate with the power line attached. The existing patent document discloses nothing about battery arrangement and this can imply its lack of deep consideration for portability and miniaturization. Moreover, a Bluetooth chip is neither able to drive the display nor to control the inflating/deflating components, and cannot be used as a controller unless the circuitry is redesigned. In other words, the improvement of hardware is very challenging. Furthermore, when the application US20060111639A1 was filed in 2006, the Bluetooth technology was in its early stage of Version 2.0. At that time, Bluetooth chips were not designed for high power consumption and did not have sufficient bandwidth for transmitting and receiving data, so as not to be able to undertake additional tasks. Therefore, a person skilled in the art would not use a Bluetooth 2.0 chip for controlling tasks and would not have the motive to perform improvement for this end.

Additionally, in the art known by the inventor, a sphygmomanometer is equipped with separate modules for analysis and communication, respectively, for ensuring accurate computing and separate circuits are used to prevent signal conflicts between the analysis module and the communication module, thereby ensuring stable communication. Examples are disclosed in applications CN 209 611 120 U and CN 106 983 503 A.

**SUMMARY OF THE APPLICATION**

In view of the shortcomings of the existing art, the present disclosure provides an electronic sphygmomanometer with an integrated air pump and a control method thereof. The electronic sphygmomanometer of the present disclosure uses a Bluetooth chip to perform control and adopts step-up measurement, so as to address one or more technical issues in the art.

The present disclosure provides an electronic sphygmomanometer that is in two-way data connection with a mobile terminal through wireless communication. Specially, the disclosed electronic sphygmomanometer has its display unit deposited on the mobile terminal.

Preferably, the electronic sphygmomanometer of the present disclosure comprises a main body for executing blood-pressure measurement and a sleeve for being wound around a body portion of a measurement subject for collecting signals, wherein the main body and the sleeve are detachably coupled to each other. The sphygmomanometer main body mainly comprises a casing, a circuit board, the integrated air pump, a battery, and a switch deposited on the surface of the main body. The circuit board integrates a master controller, a driving unit, a detection unit, and a charging unit.

More preferably, the switch deposited on the surface of the sphygmomanometer main body merely serves to turn on/off the sphygmomanometer and switch between Bluetooth working modes. Except for the switch, all the operational units of the sphygmomanometer related to blood-pressure measurement are provided on a mobile terminal that is in wireless communication with the sphygmomanometer. All operational instructions for the sphygmomanometer are given through the mobile terminal bound with the sphygmomanometer through wireless communication. The operational instructions may include a start-to-measure instruction, a stop-measuring instruction, and/or a dynamic-blood-pressure-measurement command.

Preferably, the sleeve wraps an inflatable/deflatable bladder that is in gas connection with the integrated air pump. The sleeve may be in the form of an arm band or a wrist band. Specially, the bladder performs pressure control operations related to blood-pressure measurement on the wrist and/or arm of the measurement subject based on movements of the integrated air pump related to gas control.

Further, the sleeve has a first section that is mechanically coupled to the sphygmomanometer main body and a second section that is properly elastic to fit the wrist and/or arm of the measurement subject. The second section is formed integratedly with the first section but is mechanically isolated from the sphygmomanometer main body.

Preferably, the master controller of electronic sphygmomanometer at least comprises Bluetooth chips for control, computing and communication as well as a Bluetooth control circuit. The Bluetooth chip integrates a Bluetooth communication module capable of wireless communication and a Bluetooth control module capable of computing and control. It is to be noted that the description "the Bluetooth chip integrates a Bluetooth communication module capable of wireless communication and a Bluetooth control module capable of computing and control" refers to that the Bluetooth control circuit on the Bluetooth chip in the master controller has both functions of communication and computing. In other words, the Bluetooth chip is functionally divided into a Bluetooth communication module for wireless communication and a Bluetooth control module for computing and control, instead of a Bluetooth communication module and a Bluetooth control module that are formed by multiple chips.

Further, the Bluetooth communication module is in two-way data connection with a mobile terminal, so as to receive instructions related to blood-pressure measurement and issued by the mobile terminal and send the instructions to the Bluetooth control module. The Bluetooth control module serves to analyze the received instructions related to blood-pressure measurement and performs control operations and data processing tasks related to blood-pressure measurement based on blood-pressure measurement programs and blood-pressure measurement algorithms, so as to obtain measurement results.

Specifically, the instructions related to blood-pressure measurement at least include a first instruction for starting blood-pressure measurement and a second instruction for stopping the progress of blood-pressure measurement. Preferably, a third instruction for switching blood-pressure measurement modes may be included. The blood-pressure measurement modes may include timed blood-pressure measurement, dynamic blood-pressure measurement, multi-location blood-pressure measurement, etc. Control operations related to blood-pressure measurement at least include an inflation/pressurization control operation and a deflation/depressurization control operation acting on the driving unit.

Preferably, in the first measurement stage, the Bluetooth control module is not yet able to figure out the blood pressure value according to a sequence of the dynamic air pressure values collected by the pressure sensor, and the Bluetooth communication module regularly sends dynamic air pressure values currently in the sleeve collected by the pressure sensor to the mobile terminal. In the second measurement stage, Bluetooth communication module regularly sends the dynamic air pressure values to the mobile terminal while the integrated air pump continuously inflates/pressurizes the sleeve. The pressure sensor keeps performing measurement until the Bluetooth control module is able to figure out the blood pressure value based on the pressure value sequence. The Bluetooth communication module associates the blood pressure value, figured out by the Bluetooth control module, with the heart rate to form a Bluetooth data set and sends the Bluetooth data set to the mobile terminal. The Bluetooth control module issues a stop-measuring instruction.

Preferably, the driving unit of the sphygmomanometer at least comprises an inflation driver and the integrated air pump as well as a driving circuit. The inflation driver drives and/or switches and/or stops working modes of the integrated air pump based on the measurement command issued by the Bluetooth control module. The integrated air pump is configured to work alternatively with different working modes based on variation of current polarity of a driving circuit connected thereto. Further, the working modes of the integrated air pump include a first working mode in which the sleeve is inflated for pressurization by a motor driven into forward rotation and a second working mode in which the sleeve is deflated for depressurization by the motor driven into reverse rotation. Specially, the airflow channel of the integrated air pump is reversible. In the first working mode, the airflow channel is an inflation channel; and in the second working mode, the airflow channel is a deflation channel.

More preferably, the inflation driver is of a negative feedback control design. The driving circuit performs pulse-width modulation (PWM) on the integrated air pump using the PID control algorithm so as to achieve stable inflation.

Preferably, the detection unit of the sphygmomanometer comprises a pressure sensor and an AD converter. The pressure sensor is installed inside the sleeve for collecting pulse pressure data of the user and outputting analog voltage signals. The analog voltage signals are converted into digital signals by the AD converter and sent to the Bluetooth control module for analysis and computing.

Preferably, the charging unit of the sphygmomanometer comprises a data interface, a battery, and a battery management module as well as an interface circuit and a charging circuit. The data interface serves to charge the battery of the sphygmomanometer through the interface circuit and the charging circuit. The battery serves to power components of the sphygmomanometer. The battery management module serves to provide the battery with reliable over-charge and/or over-discharge protection, over-current and/or over-heat protection, and fault diagnosis protection.

Preferably, when the battery fails to power the modules due to a breakdown, the driving circuit drives the integrated air pump to immediately enter the second working mode so as to release the air pressure in the sleeve and stop working.

Specially, the data interface may further be mechanically and/or signally connected to other health measuring equipment so as to provide enriched health monitoring functions. The data interface is preferably a Type-C interface or a magnetic Hall switch.

Preferably, during blood-pressure measurement, the Bluetooth chip performs control through the process below.

First, the Bluetooth control module, in response to the first instruction, issues initialization instructions to the components and/or units related to blood-pressure measurement, and, upon successful completion of initialization tasks performed on the components and/or units related to blood-pressure measurement, issues an instruction related to pressure control to the driving unit.

After that, the inflation driver of the driving unit, in response to the instruction related to pressure control and issued by the Bluetooth control module, performs operations related to pressure control on the integrated air pump by altering the current polarity of the driving circuit.

The instruction related to pressure control may be a pressurization instruction and/or a depressurization instruction. The operations related to pressure control include changing the current polarity of the driving circuit to a first polarity and/or a second polarity so as to drive and/or switch and/or stop the working mode of the integrated air pump.

During inflation/pressurization, the pressure sensor sends a sequence of the collected pressure values, which are discrete but increase over time, to the Bluetooth control module for the Bluetooth control module to perform data processing on the pressure value sequence using a peak-detection algorithm and a step-up blood-pressure measurement algorithm so as to obtain a heart rate value and a blood pressure value.

Specially, during pressurization for measurement, when the Bluetooth control module is not yet able to figure out the blood pressure value according to the pressure value sequence collected by the pressure sensor, the Bluetooth communication module regularly sends the dynamic air pressure value currently in the sleeve collected by the pressure sensor to the mobile terminal.

While the Bluetooth communication module regularly sends the dynamic air pressure values to the mobile terminal, the integrated air pump keeps inflating the sleeve and the pressure sensor keeps measuring pressure, until the Bluetooth control module becomes able to figure out the blood pressure value based on the pressure value sequence. Then the Bluetooth communication module associates the blood pressure value, figured out by the Bluetooth control module through calculation, to the heart rate to form a Bluetooth data set, and sends the Bluetooth data set to the mobile terminal. The Bluetooth control module thus issues a stop-measuring instruction.

Preferably, in the present disclosure, the Bluetooth chips for control, computing and communication are connected to other units as detailed below.

The RX lead and the TX lead of the Bluetooth chip are connected to the interface circuit. When the data interface is connected to an external power source, the RX lead and the TX lead are used to perform level shifting during charging.

When the data interface is connected to other health measuring equipment, the RX lead and the TX lead are used to build communication with the connected equipment.

Further, the interface circuit introduces electricity into the charging circuit, and the charging circuit charges the battery. The charging circuit is connected to the Bluetooth chip through the PROG lead and the CHG lead of the Bluetooth chip, so that the charging circuit has a constant charging current under the control of the Bluetooth chip and the Bluetooth chip can thus monitor the charging circuit.

The Bluetooth chip uses its SCK lead and DATA lead to have connection with the measurement circuit, and is then connected to the pressure sensor, thereby learning variation of the air pressure during step-up measurement.

The Bluetooth chip is connected to the driving circuit at its PWM lead, so as to drive the integrated air pump to inflate and/or deflate the sleeve, and stably control the speed of inflation.

The RF lead and the GND lead of the Bluetooth chip are connected to an external antenna circuit so as to provide basic hardware required by radio transmission and/or reception for the Bluetooth BLE.

The XTALIN lead and the XTALON lead of the Bluetooth chip are connected to an external crystal oscillator, so as to output a precise and stable frequency signal as the clock source for the Bluetooth chip.

More preferably, the Bluetooth chip may have its spare leads connected to an attached management circuit of the connected health measuring equipment, and have the Bluetooth BLE connected to a smartphone APP, so as to form an attached measurement management system.

The electronic sphygmomanometer of the present disclosure adopts step-up measurement for blood-pressure measurement, and the underlying principle is described below:

The pressure sensor records the original pulse wave of the measurement subject during the step-up process, and then the control module filters the original pulse wave to recognize its peak point and trough point so as to get the corresponding amplitude value. Afterward, curve fitting is performed according to the amplitude value sequence so as to produce a pulse wave amplitude curve. Therein, the pressure value corresponding to the maximum amplitude value or the peak point of the pulse wave is the mean arterial pressure. Subsequently, an amplitude coefficient method is used to derive the systolic blood pressure amplitude value and the diastolic blood pressure amplitude value. At last, the corresponding static pressure values determined through curve fitting are the corresponding systolic blood pressure and diastolic blood pressure.

During measurement, the pressure sensor collects a sequence of the dynamic air pressure values, which are discrete but increase over time. The air pump first performs inflation/pressurization until it is possible to detect the pulse wave, and the pressure sensor starts to record the air pressure waveform. During the ongoing inflation/pressurization performed by the air pump, when the maximum pulse wave amplitude value appears, the mean arterial pressure is determined. Then the mean arterial pressure is used to determine the diastolic blood pressure and the systolic blood pressure, thereby obtaining the values of the diastolic and systolic blood pressure before the air pump performs depressurization to terminate measurement.

The present disclosure further provides a control method of an electronic sphygmomanometer, which can be applied to the aforementioned electronic sphygmomanometer with an integrated air pump. The control method comprises the following steps:

Step S1: building two-way data transmission between the electronic sphygmomanometer and the mobile terminal through Bluetooth broadcast;

Step S2: in response to a start-to-measure instruction given by a user through a mobile terminal, executing an initialization task for at least one component related to blood-pressure measurement and issuing an instruction related to pressure control upon successful completion of the initialization task;

Step S3: based on the instruction related to pressure control, executing pressure control for the integrated air pump, and processing pressure data collected by the pressure sensor so as to obtain a blood pressure value and a heart rate value;

Step S31: during pressurization, when signals collected by the pressure sensor are not sufficient for determine valid pulse data, having the Bluetooth communication module regularly send the current air pressure value determined by the pressure sensor to the mobile terminal; and

Step S32: when the signals collected by the pressure sensor become sufficient for determine the valid pulse data, having the Bluetooth communication module form a Bluetooth data set associated with the pulse and send the Bluetooth data set to the mobile terminal,

Therein, the Bluetooth data set includes blood pressure data and heart rate data collected parallelly, and preferably blood pressure data and heart rate data collected simultaneously;

Therein, the blood pressure data in the Bluetooth data set include a mean arterial pressure, a diastolic blood pressure, and a systolic blood pressure determined by the control module of the Bluetooth chip of the sphygmomanometer through calculation.

Preferably, the mean arterial pressure, the diastolic blood pressure, and the systolic blood pressure are sent to the mobile terminal in the same Bluetooth data set or in a later Bluetooth data set.

Preferably, in the process of forming the pulse-associated Bluetooth data set, the pressure sensor remains measuring the pressure. At this time, the pressure data collected by the pressure sensor are stored into the cache of the Bluetooth chip to be used in the next CPU cycle related to "Peak and Trough" determination.

Step S4: issuing a stop-pressurizing instruction in response to a measurement-end event.

The measurement-end events include a stop-measuring instruction actively issued by the master controller based on measurement-completion conditions and/or a stop-measuring instruction made by a user at the mobile terminal. Therein, a stop-measuring instruction given by the user through the mobile terminal has priority over a stop-measuring instruction actively issued by the master controller.

Preferably, the measurement-completion conditions include: whether the Bluetooth control module has figured out the diastolic blood pressure, the systolic blood pressure, and the mean arterial pressure, whether inflation/pressurization performed by the integrated air pump has made the pressure exceed the predetermined maximum pressure value, and whether the duration of measurement has exceeded the predetermined maximum duration. When any of the measurement-completion conditions is satisfied, it is determined that measurement is to be ended.

Preferably, calculation for determining the mean arterial pressure, the diastolic blood pressure and/or the systolic blood pressure may be conducted at the APP.

Preferably, the mobile terminal may turn on/off the sphygmomanometer through transmission of Bluetooth signals, and switch the sphygmomanometer among different blood-pressure measurement modes, such as a normal mode where a single cycle of blood-pressure measurement is done or a dynamic mode where several successive cycles of blood-pressure measurement are done in a certain time period.

Further, the mobile terminal may further display and analyze the blood pressure data, and in the event of any abnormality, such as a high blood pressure, a low blood pressure, a low heart rate, a high heart rate, or a measurement error, the mobile terminal displays an alerting prompt immediately.

Furthermore, the mobile terminal may perform health data management by incorporating data obtained at other health monitoring equipment, and may further provide cloud data services so as to enable the measurement subject or people involved, such as his/her relatives, to review or share the data.

According to the previous description it is clear that the electronic sphygmomanometer of the present disclosure represents improvements in the structure of the main body and the control method over the existing art.

First, as to the structure of the main body, the present disclosure uses an integrated air pump that switches between a first working mode and a second working mode depending on current polarity, wherein the integrated air pump performs inflation or deflation in the way that the motor rotates forward or reversely. In addition, the electronic sphygmomanometer of the present disclosure uses the step-up measurement method so that deflation speed of the air pump is not a factor to be controlled, thereby eliminating the need of a solenoid valve and a speed-set air evacuation valve as required in the step-down measurement method. The step-up measurement method is a kind of oscillometric method. It is well recognized that the oscillometric method is currently the most precise non-invasive blood-pressure measurement method, which means measurement precision of the disclosed electronic sphygmomanometer is ensured. On this basis, the present disclosure reduces redundant components while ensuring measurement precision as compared to existing electronic sphygmomanometers. This not only decreases manufacturing costs, but also simplifies control of inflation/deflation performed by the air pump during blood-pressure measurement, thereby improving the efficiency of blood-pressure measurement. Moreover, such a simple control process significantly reduces the computing load imposed on the control unit of the sphygmomanometer master, and in turn lower power consumption of the sphygmomanometer.

Then is about the control method. The electronic sphygmomanometer of the present disclosure pioneeringly employs a single Bluetooth chip to integrate the combined functions of the MCU chip and the Bluetooth chip in an existing electronic sphygmomanometer. Further, with reasonable design of the leads of the Bluetooth chip, the communication module and the control module of the Bluetooth chip can operate for measurement without interfering with each other. During measurement, the mean arterial pressure, the systolic blood pressure, and the diastolic blood pressure as well as the heart rate are all determined by the Bluetooth control module through computing. When the Bluetooth control module performs computing, the Bluetooth communication module transmits just little or even no data. By incorporating processing, control and analysis conventionally performed by an MCU chip into a single Bluetooth chip through hardware design, the present disclosure makes full use of the computing power of the Bluetooth chip it is equipped for communication, and achieves more reasonable resource allocation in the sphygmomanometer, so as to reduce manufacturing costs while improving measurement efficiency of the sphygmomanometer.

## BRIEF DESCRIPTION OF THE DRAWINGS

FIG. 1 is a perspective view of an electronic sphygmomanometer of the present disclosure;

FIG. 2 is an exploded view of the electronic sphygmomanometer of the present disclosure;

FIG. 3 is a block diagram showing hardware connection of the electronic sphygmomanometer of the present disclosure;

FIG. 4 is a circuit diagram showing circuit connection of Bluetooth control chip in the electronic sphygmomanometer of the present disclosure; and

FIG. 5 are graphs wherein curve fitting underlying step-up measurement of the present disclosure is illustrated.

Drawing references

| 1: | Main Body of Sphygmomanometer | 101: | Casing |
|---|---|---|---|
| 2: | Sleeve | 102: | Switch |
| 3: | Bluetooth Chip | 103: | Data Interface |
| 4: | Driving Circuit | 104: | Circuit Board |
| 5: | Integrated Air Pump | 105: | Detection Air Channel |
| 6: | Measurement Circuit | 106: | Detection Air Channel Connector |
| 7: | Pressure Sensor | 107: | Air Valve |
| 8: | Interface Circuit | 201: | First Section of Sleeve |
| 9: | Other Health Measuring Equipment | 202: | Second Section of Sleeve |
| 10: | Charging Circuit | 203: | Detection Air Channel Port |
| 11: | Battery | 204: | Air Valve Port |

## DETAILED DESCRIPTION OF THE APPLICATION

It is to be noted that when a component is described as being "fixed to" or "provided on" another component, the component may directly contact the second component or there may be an intermediate component situated therebetween. When a component is deemed as being "connected to" another component, it may be directly connected to the second component or there may be an intermediate component situated therebetween. In the disclosure, the terms such as "vertical", "horizontal", "up", "down", "left" or "right" are only illustrative but not intended to limit possible implementations.

In addition, an ordinal number such as "first" or "second" is merely descriptive and shall not be construed as indicating or implying relative importance or suggesting the quantity of the described technical feature. Thereby, description of a feature with the term "first" or "second" may indicate or imply inclusion of at least one said feature. In the disclosure, unless specifically indicated in the context, where "plural" means that there are a number of at least two, such as two or three.

Unless otherwise defined, all the technical and scientific terms used in the disclosure shall have the definitions as commonly known by people skilled in the art. These terms used herein are intended to describe particular implementations of the present disclosure and by no means limiting. In the disclosure, where the term "and/or" is used in a list of items, it refers to any or all combinations of one or more of the listed items.

The electronic sphygmomanometer of the present disclosure represents improvements in the structure of the main body and the control method over the existing art. As to the structure of the main body, the present disclosure uses an integrated air pump that performs both inflation and deflation depending on current polarity, and uses the step-up measurement method so that deflation speed of the air pump is not a factor to be controlled, thereby eliminating the need of a solenoid valve and a speed-set air evacuation valve. As to the control method, the electronic sphygmomanometer of the present disclosure pioneeringly employs a single Bluetooth chip to integrate the combined functions of the MCU chip and the Bluetooth chip in an existing electronic sphygmomanometer. The details will be described in the following paragraphs.

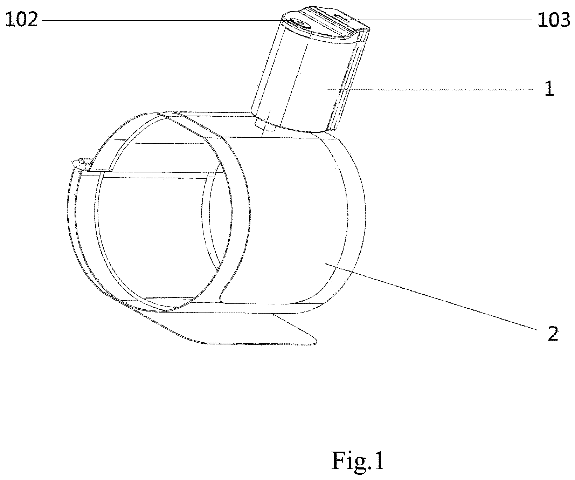

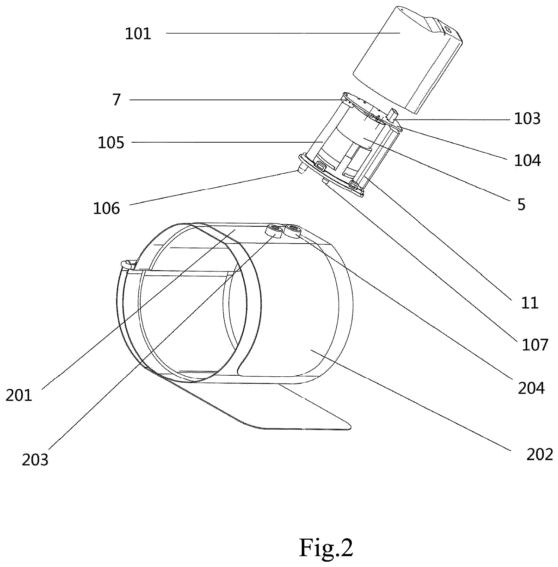

According to a preferred mode, as shown in FIG. 1, the electronic sphygmomanometer of the present disclosure comprises a main body 1 and a sleeve 2 that are detachably connected. The main body 1 of the sphygmomanometer is in wireless communication with a mobile terminal, and is configured to perform operations related to blood-pressure measurement in response to instructions from the mobile terminal. The sleeve 2 is preferably an arm band or a wrist band that are interchangeable for measurement performed at different body positions. Further, as shown in FIG. 2, the sleeve 2 is composed of a first section 201 that is rigid enough to bear the main body 1 of the sphygmomanometer and a second section 202 that is elastic so as to tightly fit the wrist and/or arm of a measurement subject when tensioned. The first section 201 is coupled to the main body 1 of the sphygmomanometer, and the second section 202 is not in contact with the main body 1 of the sphygmomanometer. Specially, the sleeve 2 contains therein an inflatable/deflatable bladder (not shown) for pressing or releasing the wrist and/or the arm of a measurement subject.

According to a preferred mode, as shown in FIG. 1 and FIG. 2, the main body 1 of the sphygmomanometer includes a casing 101 containing therein a data interface 103, a circuit board 104, a pressure sensor 7, a detection air channel 105, and a battery 11. It is envisagable that there are more necessary supporting and/or connecting components in the main body 1 of the sphygmomanometer. Since these components are merely basic and common mechanical structures and not where the essence of the present disclosure lies, detailed description of them is herein omitted. Preferably, the main body 1 of the sphygmomanometer is provided with a switch 102. The switch 102 merely serves to turn on/off the sphygmomanometer and switch between Bluetooth working modes. Except for the switch 102, all the operational units of the sphygmomanometer related to blood-pressure measurement, such as those for giving the start-to-measure instruction, the step-measuring instruction, and the measurement-mode-switching instruction, are all provided on a mobile terminal that is in wireless communication with the sphygmomanometer. Thereby, the user can operate the mobile terminal to give the instructions.

According to a preferred mode, as shown in FIG. 1 and FIG. 2, in the electronic sphygmomanometer of the present disclosure, the data interface 103 is deposited on the circuit board 104. The data interface 103 is used to not only connect an external power source for charging the battery 11, but also connect other health measuring equipment 9 for achieving more versatile and more comprehensive health measurement functions. Further, the pressure sensor 7 is also deposited on the circuit board 104. A detection air pipe (not shown) extends outward from the pressure sensor 7 along the detection air channel 105. The detection air channel 105 is located at the lateral of the integrated air pump 5 and extends to the outside of the casing 101 with a detection air channel connector 106. Corresponding, a detection air channel port 203 matching the detection air channel connector 106 is formed at the surface of the first section 201 of the sleeve 2 so that the detection air pipe of the pressure sensor 7 can extend into the sleeve 2 where it collects pressure data. The circuit board 104 is further mechanically and communicatively connected to the integrated air pump 5. The air valve 107 of the integrated air pump 5 is engaged with the air valve port 204 in the second section 202 of the sleeve 2 to form inflation and deflation channels. In addition to these mechanical structures, the circuit board 104 integrates a master controller, a driving unit, a detection unit, and a charging unit so as to enable the electronic sphygmomanometer to accomplish its blood-pressure measurement tasks.

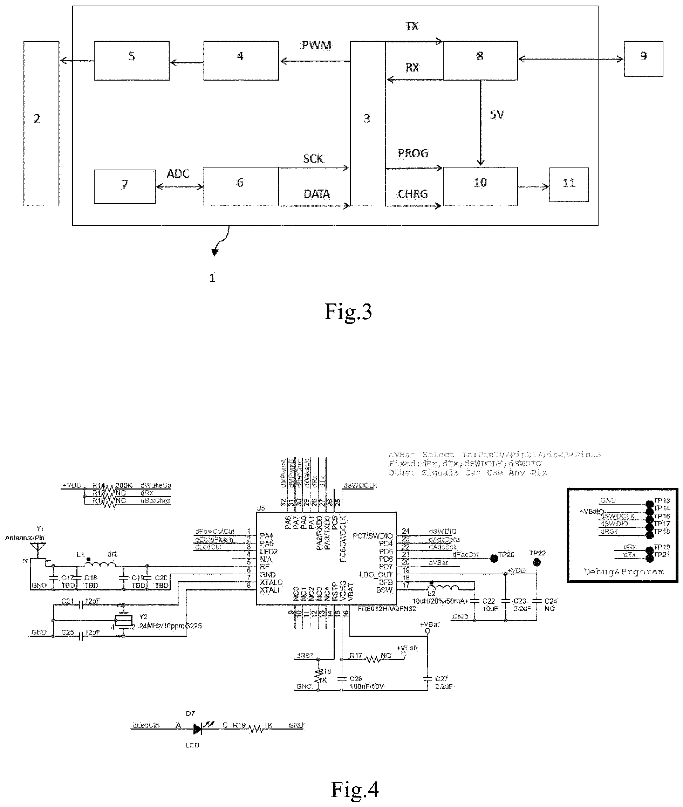

According to a preferred mode, as shown in FIG. 3, in the electronic sphygmomanometer of the present disclosure, the master controller comprises an FR8012HA low-power Bluetooth 5.1 chip and a Bluetooth control circuit. The Bluetooth chip 3 comprises a Bluetooth communication module and a Bluetooth control module. The Bluetooth control module uses the step-up blood-pressure measurement algorithm to perform computing on the air pressure digital signal values collected by the pressure sensor 7 during inflation and then converted by the AD converter, thereby obtaining the blood pressure values and the heart rate value of the measurement subject. The blood pressure values include values of the systolic blood pressure, the diastolic blood pressure, and the mean arterial pressure. The Bluetooth communication module receives a start-to-measure instruction or a stop-measuring instruction given by the user through the mobile terminal and forwards the instruction to the Bluetooth control module. The Bluetooth communication module also serves to send the blood pressure result data, obtained by the Bluetooth control module through computing, to the mobile terminal.

According to a preferred mode, as shown in FIG. 3 and FIG. 4, the RX lead (Pin28) and the TX lead (Pin27) of the Bluetooth chip 3 are connected to the interface circuit 8. When the data interface 103 is connected to an external power source, the RX lead (Pin28) and the TX lead (Pin27) are used to perform level shifting during charging. When the data interface 103 is connected to other health measuring equipment 9, the RX lead (Pin28) and the TX lead (Pin27) are used to build communication with the connected equipment.

The interface circuit 8 introduces 5V power into the charging circuit 10 so that the charging circuit 10 can charge the battery 11. The charging circuit 10 is connected to the Bluetooth chip 3 through the leads Pin2, Pin20, Pin30 of the Bluetooth chip 3, so as to detect whether a charger is connected to the charging circuit 10 or detect the charging state and the charge level of the battery 11. In other words, the leads Pin2, Pin20, and Pin30 work with the charging circuit 10 to form a power source management module for managing and monitoring the charging process of the sphygmomanometer. Further, the leads Pin17, Pin18, and Pin19 are connected to the battery 11 to form a battery power system. The leads Pin15 and Pin16 of the Bluetooth chip 3 are connected to a reset circuit so as to reset the Bluetooth control module thereby preventing the Bluetooth control module from giving wrong instructions and/or performing wrong operations.

At its SCK lead (Pin22) and DATA lead (Pin23), the Bluetooth chip 3 is connected to the measurement circuit 6. The measurement circuit 6 comprises the pressure sensor 7 and an AD converter. The pressure sensor 7 collects pressure data in the sleeve 2 and uses the AD converter to covert the data into electronic signals that are then sent to the Bluetooth chip 3 through the measurement circuit 6 for the Bluetooth chip 3 to conduct subsequent data processing.

At its PWM leads (Pin31 and Pin32), the Bluetooth chip 3 is connected to the driving circuit 4 so as to control inflation and deflation of the sleeve 2 during blood-pressure measurement. Control of inflation and collection of air pressure forms a closed-cycle system. The Bluetooth control module uses a blood-pressure measurement algorithm to perform computing on the collected data so as to obtain a blood pressure value and a heart rate value. Then the Bluetooth communication module transmits the blood pressure value and the heart rate value to the APP at the smartphone where the values are input into the top-level management system.

The RF lead (Pin5) and the GND lead (Pin6) of the Bluetooth chip 3 are connected to an external antenna circuit so as to provide basic hardware required by radio transmission and/or reception for the Bluetooth BLE.

The XTALIN lead (Pin8) and the XTALON lead (Pin7) of the Bluetooth chip 3 are connected to an external crystal oscillator so as to output a precise and stable frequency signal as the clock source for the Bluetooth chip 3.

According to a preferred mode, the leads Pin1, Pin27, Pin28, and Pin29 of the Bluetooth chip 3 may be connected to other health measuring equipment 9 to form an attached management circuit. Moreover, the data link of the Bluetooth BLE may be connected to the mobile terminal to form a measurement attached management system, so as to realize detection of connection status, power control and communication control for attached equipment. Further, the attached health monitoring equipment may receive instructions for management and control from the top-level management system through the data link of the Bluetooth BLE, and may have interaction of the measurement data with the mobile terminal.

According to a preferred mode, the driving unit in the present disclosure comprises an inflation driver, the integrated air pump 5, and the driving circuit 4. The inflation driver is preferably a motor driving chip modeled FM8301 or FM118S, and serves to receive start-to-inflate and stop-inflating instructions issued by the Bluetooth control module and drive the integrated air pump 5 to inflate and deflate the bladder in the sleeve 2. Further, the inflation driver performs negative feedback control. For example, the preset target speed for pressurization Ps is 3 - 5mmHg/s. Depending on how tightly the measurement subject wears the sleeve 2, there is a fluctuant difference between the actual pressurization speed $Pi$ and the preset target speed $Ps$, which means that $Pi-Ps=\Delta P$ is changing. At this time, the inflation driver selects one from plural preset pressurization curves so that $\Delta P$ is in a reasonable threshold range. When the inflation driver finds $\Delta P$ that is in the reasonable threshold range, it then selects the corresponding pressurization curve and changes the pressurization speed by means of PWM, thereby achieving curve pressurization.

According to a preferred mode, the sphygmomanometer of the present disclosure employs the integrated air pump 5 having two working modes, namely inflation and deflation. For example, the air pump described in China Patent Publication No. CN217682211U is in its inflation working mode when the motor rotates forward and is in its deflation working mode when the motor rotates reversely. The air pump has a reversible airflow channel. When the motor rotates forward, with transmission performed by driving members and linkage, the bladder in the air pump performs expansion-contraction movement to press air into a buffer chamber and the air then flows out through an inflation port. At this time, the airflow channel acts as an inflation channel. When rotating reversely, the motor activates the driving members and linkage to drive the exhaust mechanism to unblock the deflation port. This allows the air flow to run from the inflation port to the deflation port and then exhaust to atmosphere. In this case, the airflow channel acts as a deflation channel. To control forward/reverse rotation of the motor of the integrated air pump 5, it is necessary to reverse the current polarity of the driving circuit 4 where the motor is situated. Therefore, the present disclosure further provides a driving circuit 4 that is electrically connected to the Bluetooth chip 3. The driving circuit 4 mainly comprises an inflation driver, the integrated air pump 5, and related circuit elements. When receiving an initialization instruction issued by the Bluetooth chip 3, the motor driving chip initializes the integrated air pump 5 so as to activate all the components in the air inlet channel. When receiving a pressurization instruction issued by the Bluetooth chip 3, the motor driving chip puts the circuit current into the first polarity so that the motor in the integrated air pump 5 rotates forward, and the integrated air pump 5 operates in its first working mode for inflating the bladder in the sleeve for pressurization. Similarly, when receiving a stop-pressurizing instruction issued by the Bluetooth chip 3, the motor driving chip puts the circuit current into the second polarity so that the motor in the integrated air pump 5 rotates reversely, and the integrated air pump 5 operates in its second working mode for deflating the bladder in the sleeve 2 for depressurization.

According to a preferred mode, in the disclosed sphygmomanometer, the charging unit comprises a data interface 103, a battery 11, and a battery management module. The data interface 103 is mechanically and/or electrically coupled to the Bluetooth chip 3, and serves to charge the battery 11. It is further connected to other health monitoring equipment. The data interface 103 is of the USB specifications, and may be, for example, a mini-USB, a micro-USB, or a Type-C interface, and more preferably a Type-C interface.

According to a preferred mode, when the data interface 103 of the main body 1 of the sphygmomanometer is connected to other health monitoring equipment or health monitoring modules, the mobile terminal in data connection with the main body 1 of the sphygmomanometer is switched to the corresponding health detection mode. Specifically, with expansion enabled by the data interface 103, the sphygmomanometer may be further able to measure body temperature, blood contents, and/or urine contents. In this case, the master controller in the sphygmomanometer main body is only used to start and/or stop measurement, and receive and transmit measurement data.

The battery 11 powers various components of the sphygmomanometer. The battery management module provides the battery 11 with reliable over-charge and/or over-discharge protection, over-current and/or over-heat protection, and fault diagnosis protection. The main functions rely on a battery charging management chip. The battery charging management chip is preferably a TP4054 chip. The battery charging management chip TP4054 serves to transform, distribute, and detect the electrical power required various components of the sphygmomanometer, and may further be able to recognize the power-supply amplitude value of the Bluetooth chip 3, so as to generate the corresponding short rectangular wave to drive the post circuit to output power.

According to a preferred mode, in the disclosed sphygmomanometer, the detection unit comprises a pressure sensor 7, an AD converter, and a measurement circuit 6. The pressure sensor 7 is configured in the sleeve 2 and serves to collect pulse pressure data of a user to form original pulse wave signals. The original pulse wave signals are sent to the AD converter. The AD converter convers the analog original pulse wave signals into digital signals and sends the digital signals to the Bluetooth chip 3. It is to be noted that the signals collected by the pressure sensor 7 are mixed signals composed of pulse wave signals and static pressure signals, and may also contain some high-frequency interference and DC/low-frequency components coming from the outside. The static pressure signals are low-frequency signals having a frequency smaller than or equal to 0.04Hz. The pulse wave signals usually have a frequency of about 1Hz. Therefore, the mixed signals have to be filtered and amplified by the Bluetooth control circuit to become pulse wave signals that are purer before they can be sent to the Bluetooth control module for calculation of blood pressure data.

As another preferred mode, in the present disclosure, the sleeve 2 may be made as a wrist band or an arm band for a user to choose according to practical needs. The sphygmomanometer of the present disclosure is further able to sense where sleeve 2 is and run the corresponding measurement program. Due to the dimensional difference between a wrist and an arm, the inflation/pressurization rates for the wrist band and the arm band are different. To this end, the control module of the Bluetooth chip 3 may further determine whether the sleeve 2 is currently worn by the user around his/her wrist or arm according to the current inflation/pressurization rate, thereby correctly determine whether the wrist measurement program or the arm measurement program shall be executed. The wrist measurement program and the arm measurement program implement the same control process. The only difference between the two programs is about selection of coefficients for normalization of the blood pressure data.

According to a preferred mode, in the electronic sphygmomanometer of the present disclosure, during a cycle of blood-pressure measurement, the control process of the Bluetooth chip is as below.

At Step S1, the Bluetooth communication module receives a first instruction made by a user at the mobile terminal that requires start of blood-pressure measurement and sends the instruction to the Bluetooth control module.

At Step S2, the Bluetooth control module, in response to first instruction, gives initialization instructions to the detection unit, the driving unit, and the master controller where the Bluetooth control module itself is in, and, upon successful completion of the initialization tasks at all the relevant units, issues a pressurization instruction to the driving unit. Meanwhile, the Bluetooth control module issues a start-to-measure instruction to the detection unit and powers the measurement circuit 6.

At Step S3, the inflation driver of the driving unit, in response to the pressurization instruction from the Bluetooth control module, puts the current polarity of the driving circuit 4 into the first polarity so as to drive the integrated air pump 5 to operate in its first working mode where it performs inflation/pressurization on the sleeve.

Therein, the inflation driver performs PWM on the integrated air pump 5 using a PID control algorithm so as to control the inflation speed of the integrated air pump 5.

At Step S4, the pressure sensor 7, when powered on, collects the original pressure data in the sleeve and sends the original pulse wave signal in the form of analog signals to the AD converter. The AD converter convers the original pulse wave signals into digital signals and sends the converted signals to the Bluetooth control module.

Therein, the original pulse wave signals form a pressure value sequence being discrete but increasing over time.

At Step S5, the Bluetooth control module recognizes the pulse wave peak point in the pressure value sequence of the original pulse wave signals, and determines the heart rate according to the number of the peak points appears in a preset time period. The heart rate value is cached in the cache of the Bluetooth chip 3 before being sent out subsequently.

At Step S6, the Bluetooth control module process the digital original pulse wave signals and removes noises like baseline drift by combining Butterworth high-pass filtering and low-pass filtering, so as to extract purer pulse wave signals as the dynamic pressure value. A Butterworth low-pass filter algorithm is used to extract the DC component in the signals as the static pressure reference value in the sleeve.

At Step S7, the Bluetooth control module controls the integrated air pump 5 to continuously perform pressurization, and when it is possible to detect an effective pulse wave, the pressure sensor starts to record the air pressure waveform. Therein, the effective pulse wave may be an empirical value where a signal fluctuation exceeding 1.5mmHg.

At Step S8, the Bluetooth control module detects peaks and troughs of the processed pulse wave signals by searching them one by one so as to determine the ascending and descending process of the sequence and recognize peak points and trough points. The difference between a peak point and a trough point is the amplitude value. The peak points, the trough points, the amplitude value points, and the pulse number are then mapped to generate a pulse wave amplitude value curve.

At Step S9, the Bluetooth control module recognizes the pulse wave amplitude value curve by taking the static pressure value corresponding to the site where the amplitude value is greatest as the mean arterial pressure, and figuring out the diastolic blood pressure and the systolic blood pressure based on the empirical coefficients among the mean arterial pressure, the diastolic blood pressure, and the systolic blood pressure using the amplitude value coefficient method.

During pressurization, when the Bluetooth control module is not yet able to figure out the blood pressure value according to the dynamic pressure value, or, when the Bluetooth control module is not yet able to figure out the mean arterial pressure, the diastolic blood pressure, and the systolic blood pressure according to pulse wave amplitude value curve, the Bluetooth communication module sends the dynamic air pressure value currently in the sleeve as determined by the pressure sensor 7 to the mobile terminal regularly.

The term "regularly" means that when the pressure sensor 7 has not detected a pulse, the Bluetooth communication module sends the air pressure value currently in the sleeve to the mobile terminal at an interval of 1s, and after the pressure sensor 7 starts to detect a pulse, the Bluetooth communication module sends the air pressure value currently in the sleeve to the mobile terminal according to the pulse interval, which is about 1s.

During the period that the Bluetooth communication module regularly sends the dynamic air pressure values to the mobile terminal, the pressure sensor 7 keep measuring the pressure. At this time, the pressure data collected by the pressure sensor 7 are cached in the cache of the Bluetooth chip 3 to be used in the next CPU cycle related to "Peak and Trough" determination.

At Step S10, as the integrated air pump 5 keeps performing inflation, when the Bluetooth control module finally becomes able to figure out the blood pressure value based on the pulse wave amplitude value curve, the Bluetooth communication module combines the blood pressure value figured out by the Bluetooth control module, associated with the heart rate, to form a Bluetooth data set and sends the Bluetooth data set to the mobile terminal. Associating the data to the heart rate is achieved by recording and transmitting the blood pressure value and the heart rate value in the same time frame together.

At Step S11, the Bluetooth communication module sends the heart rate value and the blood pressure value to the mobile terminal. This satisfies the measurement-end recognition condition and thereby triggers a measurement-end event. At this time, the Bluetooth control module, in response to the establishment of the measurement-end event, issues a depressurization instruction to the driving unit.

At Step S12, the inflation driver of the driving unit, in response to the depressurization instruction, puts the current of the driving circuit 4 into the second polarity so as to control the integrated air pump 5 to operate in its second working mode where it performs deflation/depressurization on the sleeve.

Meanwhile, the Bluetooth communication module issues a stop-measuring instruction to the measurement unit so that the pressure sensor 7 and the AD converter are de-powered and stop operating, making the present cycle of blood-pressure measurement completed.

The electronic sphygmomanometer of the present disclosure adopts a step-up measurement method, and the underlying principle will be described below.

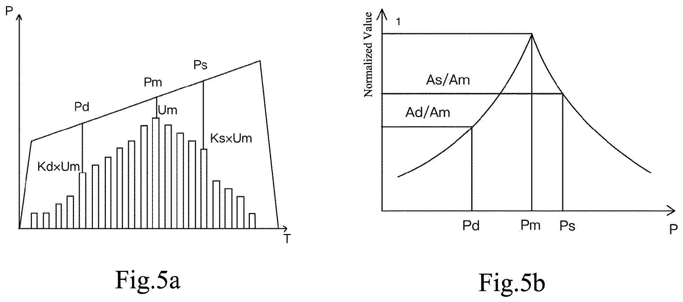

The pressure sensor 7 collects the original pulse wave of the measurement subject during the step-up process, and then the control module filters the original pulse wave to recognize the peak points and trough points so as to obtain corresponding amplitude values. According to the amplitude value sequence, curve fitting is performed to generate the pulse wave amplitude value curve in FIG. 5a. As shown, as the sleeve 2 gets inflated continuously, when the pressure P in the sleeve 2 increases to the extent that it becomes higher than the diastolic blood pressure Pd, the pulse wave increases sharply, and the amplitude value reaches the maximum at the mean arterial pressure Pm. When the pressure P in the sleeve 2 is much higher than the systolic blood pressure Ps, the pulse wave disappears. In this process, the pulse wave maximum value corresponds to the mean arterial pressure. The systolic blood pressure Ps and the diastolic blood pressure Pd are figured out using the amplitude value coefficient method and determined from the proportions of the pulse wave maximum amplitude values, respectively.

The amplitude coefficient method is also known as "normalization". As shown in FIG. 5b, it involves comparing the amplitude value of a pulse wave vibration signal with the maximum amplitude value of the signal for normalization, and recognizing the systolic blood pressure and the diastolic blood pressure by determining normalization coefficients of the systolic blood pressure and the diastolic blood pressure. Therein, As is the pulse wave amplitude value corresponding to the systolic blood pressure; Am is the amplitude of the pulse wave corresponding to the mean arterial pressure; Ad is pulse wave amplitude value corresponding to the diastolic blood pressure; As/Am is the normalized value of the systolic blood pressure Ps; Ad/Am is the normalized value of the diastolic blood pressure Pd; and Pc is the pressure in the sleeve 2. The abscissa represents that the pressure in the sleeve 2 keeps growing during inflation. As/Am=C1 and Ad/Am=C2 correspond to locations of the systolic blood pressure and the diastolic blood pressure, respectively.

With the measured pulse wave amplitude value and the corresponding static pressure, it is possible to determine the systolic blood pressure Ps, the diastolic blood pressure Pd, and the mean arterial pressure Pm. Generally, the systolic blood pressure Ps has an amplitude coefficient Ks of 0.40 - 0.75, and the diastolic blood pressure Pd has an amplitude coefficient Kd of 0.69 - 0.89. Based on the amplitude coefficient values commonly used in clinical medicine, namely Ks=0.45 and Kd=0.9, a method for determining blood pressure data can be obtained as detailed below.

Data determination of the systolic blood pressure Ps: in the ascending phrase of the pulse wave amplitude envelope during inflation, when the ratio between the amplitude Ui of a certain pulse wave and the maximum amplitude Um is Ui/Um=Ks, it is determined that the pressure currently in the sleeve is right the systolic blood pressure, i.e., Ps=Ks•Um.

Data determination of the diastolic blood pressure Pd: in the ascending phrase of the pulse wave amplitude envelope during inflation, when the ratio between the amplitude Ui of a certain pulse wave and the maximum amplitude Um is Ui/Um=Kd, it is determined that the pressure currently in the sleeve is right the diastolic blood pressure, i.e., Pd=Kd•Um.

According to the foregoing rules for determination of blood pressure data, the integrated air pump 5 first inflates the sleeve 2 to the extent that a pulse wave can be detected and then the pressure sensor 7 starts to record the air pressure waveform. In this process, the pressure value sequence collected by the pressure sensor 7 is discrete but increases over time. When the Bluetooth control module becomes able to locate the maximum of the pulse wave amplitude value in the waveform, the mean arterial pressure Pm can be determined. Then the mean arterial pressure Pm is used to determine the diastolic blood pressure Pd and the systolic blood pressure Ps. When the Bluetooth control module has figured out the diastolic blood pressure Pd and the systolic blood pressure Ps, it is time to perform depressurization to stop measuring.

It is to be noted that FIG. 5a and FIG. 5b are deduced graphs for explaining data processing implemented by the disclosed sphygmomanometer, not fit graphs obtained through experiments.

The present disclosure further provides a control method of an electronic sphygmomanometer that is applicable to the electronic sphygmomanometer with the integrated air pump as described previous. The control method comprises the following steps.

At Step S100, the sphygmomanometer and the mobile terminal have two-way data transmission relationship through Bluetooth broadcast.

The mobile terminal, when activated, can automatically find the sphygmomanometer that has been paired by searching the Bluetooth broadcast at the back end and then connect the identified sphygmomanometer.

At Step S200, the master controller of the sphygmomanometer, in response to a start-to-measure instruction given by the user through the mobile terminal, executes an initialization task on at least one component related to blood-pressure measurement, and, upon successful completion of the initialization task, issues instructions related to pressure control.

After the user gives a start-to-measure instruction at the mobile terminal, and master controller receives the start-to-measure instruction through the Bluetooth communication module, the various units of the sphygmomanometer, in response to reception of the instruction, execute initialization tasks that at least include initialization of the integrated air pump 5, initialization of the pressure sensor 7, and initialization of the inflation driver. The Bluetooth control module then issues a pressurization instruction to the driving unit when determining that the initialization tasks have been completed successfully.

At Step S201, the initialization task for the inflation driver includes initialization of its system properties, functional modules, and data structure, as well as verification and restoration of information stored in its system.

The initialization task for that integrated air pump 5 mainly includes resetting its various air-pipe components, which involves activation of these various air-pipe components so as to ensure that the integrated air pump 5 can operate in the first working mode without delay. The initialization task for the integrated air pump 5 is controlled and conducted by the inflation driver.

The pressure sensor 7 is configured to run initialization automatically when powered provided that a measurement subject puts the sleeve 2 on and a current static pressure value is obtained. Once the sensor senses effective VDD coming from the outside, its internal timing mechanism generates internal power-on reset POR, and starts a power-on initialization sequence automatically. The complete power-on process generally takes about 400us. After initialization of the pressure sensor 7, the bit DEV_RDY in the register INT_SRC in the pressure sensor 7 is set to 1.

At Step S202, the Bluetooth control module, based on the results of the initialization task or the post-initialization states of the integrated air pump 5 and the pressure sensor 7, detects whether the integrated air pump 5 or the pressure sensor 7 has any error, and issues a pressurization instruction to the driving unit when determining that the initialization task has been completed successfully.

If the Bluetooth control module confirms successful initialization after error detection, it moves to the next step. Otherwise, the Bluetooth control module issues repeated initialization instruction to the related components. When the number of repetitions of the initialization instruction reaches a predetermined value, the Bluetooth control module issues a suspension instruction to each of the related units and feeds back this situation to the mobile terminal through the Bluetooth communication module to enquire whether the sphygmomanometer is reset for a new attempt of initialization.

At Step S300, the inflation driver, based on an instruction related to pressure control from the master controller, performs pressure control on the integrated air pump. The master controller processes the pressure data collected by the pressure sensor 7 so as to obtain the blood pressure value and the heart rate value.

After all the initialization tasks have been completed, the inflation driver executes the pressurization command issued by the Bluetooth control module to drive the integrated air pump 5 to perform inflation. Meanwhile, the pressure sensor 7 sends the collected pressure data back to the Bluetooth control module for processing and computing to obtain the systolic blood pressure, the diastolic blood pressure, and the mean arterial pressure of the measurement subject.

At Step S301, the integrated air pump 5 operates in the first working mode to inflate/pressurize the bladder in the sleeve 2, and the pressure sensor 7 measures the pressure in the sleeve 2 so as to obtain the original pulse wave signals of the measurement subject.

At Step S302, the original pulse wave signals collected by the pressure sensor 7 in the form of analog voltage signals are amplified by the measurement circuit 6 and then sent to the AD converter. The AD converter converts the analog signals into digital signals and sent to the Bluetooth control module where they are used for figuring out the blood pressure.

At Step S303, the Bluetooth control module receives the original pulse wave signals collected by the pressure sensor 7 and removes noises like baseline drift by combining Butterworth high-pass filtering and low-pass filtering, so as to extract purer pulse wave signals as the dynamic pressure value. A Butterworth low-pass filter algorithm is used to extract the DC component in the signals as the static pressure reference value in the sleeve 2.

At Step S304, the Bluetooth control module detects peaks and troughs of the processed pulse wave signals by searching them one by one so as to determine the ascending and descending process of the sequence and recognize peak points and trough points. The difference between a peak point and a trough point is the amplitude value. The peak points, the trough points, the amplitude value points, and the pulse number are then mapped to generate a pulse wave amplitude value curve.

At Step S305, the Bluetooth control module recognizes the pulse wave amplitude value curve by taking the static pressure value corresponding to the site where the amplitude value is greatest as the mean arterial pressure, and figuring out the diastolic blood pressure and the systolic blood pressure based on the empirical coefficients among the mean arterial pressure, the diastolic blood pressure, and the systolic blood pressure using the amplitude value coefficient method.

At Step S306, since the pulse reflects the heart rate, during measurement of the blood pressure, the Bluetooth control module recognizes the peak points of the pulse wave in the original pulse wave signals, and figures out the heart rate according to the number of peak points appearing in a predetermined time period.

At Step S400, during pressurization, when signals collected by the pressure sensor 7 are not sufficient to determine the heart rate value, such as an empirical value with a signal fluctuation exceeding 1.5mmHg, the Bluetooth communication module regularly sends the current air pressure value determined by the pressure sensor 7 to the mobile terminal.

When the signals collected by the pressure sensor 7 are sufficient for determination of effective pulse data, the Bluetooth communication module associates the data with the pulse to form a Bluetooth data set, and sends the Bluetooth data set to the mobile terminal.

Specially, the Bluetooth data set includes blood pressure data and heart rate data collected simultaneously. The blood pressure data in the Bluetooth data set include the mean arterial pressure, the diastolic blood pressure, and the systolic blood pressure determined by the control module of the Bluetooth chip 3 of the sphygmomanometer, and the mean arterial pressure, the diastolic blood pressure, and the systolic blood pressure are sent to the mobile terminal in the same Bluetooth data set or in a later Bluetooth data set.

During the period that the Bluetooth communication module forms the pulse-associated Bluetooth data set, the pressure sensor 7 keep measuring the pressure. At this time, the pressure data collected by the pressure sensor 7 are cached in the cache of the Bluetooth chip 3 to be used in the next CPU cycle related to "Peak and Trough" determination.

At Step S500, the master controller, in response to triggering of a measurement-end event, issues a stop-pressurizing instruction to the inflation driver.

The measurement-end events include a stop-measuring instruction actively issued by the master controller based on measurement-completion conditions and/or a stop-measuring instruction made by a user at the mobile terminal. Therein, a stop-measuring instruction given by the user through the mobile terminal has priority over a stop-measuring instruction actively issued by the master controller.

Preferably, the measurement-completion conditions include: whether the Bluetooth control module has figured out the diastolic blood pressure, the systolic blood pressure, and the mean arterial pressure, whether inflation/pressurization performed by the integrated air pump 5 has made the pressure exceed the predetermined maximum pressure value, and whether the duration of measurement has exceeded the predetermined maximum duration. When any of the measurement-completion conditions is satisfied, it is determined that measurement is to be ended.

In the control process as described previously, calculation of the mean arterial pressure, the diastolic blood pressure, and/or the systolic blood pressure may be conducted at the mobile terminal. The mobile terminal is able to not only turn on/off the sphygmomanometer through Bluetooth signal transmission, but also direct the sphygmomanometer to perform different blood-pressure measurement modes, such as a dynamic blood-pressure measurement mode that involves continuous measurement in a given time period. Further, the mobile terminal may be able to display and analyze the blood pressure data, and in the event of any abnormality, such as a high blood pressure, a low blood pressure, a low heart rate, a high heart rate, or a measurement error, the mobile terminal displays an alerting prompt immediately. Alerting prompts are given when any of the following requirements is satisfied:

(1) The systolic blood pressure >145 mmHg or the systolic blood pressure <95 mmHg;

(2) The diastolic blood pressure >90 mmHg or the diastolic blood pressure <45 mmHg;

and

(3) The heart rate >105 or the heart rate <45.

According to a preferred mode, the APP at the smartphone may further conduct health data management by incorporating data obtained at other health monitoring equipment, and may provide cloud data services so that a measurement subject or relatives of a measurement subject can review and/or share the data.

It is to be noted that the interface circuit 8, the charging circuit 10, the driving circuit 4 and the measurement circuit 6 used in the present disclosure are configured in the forms that are commonly used in existing electronic sphygmomanometers, and are technical means well known to people skilled in the art. Hence, diagrams of these circuits are omitted in the disclosure.

It is to be noted that the particular embodiments described previously are exemplary. People skilled in the art, with inspiration from the disclosure of the present disclosure, would be able to devise various solutions, and all these solutions shall be regarded as a part of the disclosure and protected by the present disclosure. Further, people skilled in the art would appreciate that the descriptions and accompanying drawings provided herein are illustrative and form no limitation to any of the appended claims. The scope of the present disclosure is defined by the appended claims. The disclosure provided herein contains various inventive concepts, such of those described in sections led by terms or phrases like "preferably", "according to one preferred mode" or "optionally". Each of the inventive concepts represents an independent conception and the applicant reserves the right to file one or more divisional applications therefor.

**Claims**

1. An electronic sphygmomanometer with an integrated air pump, the electronic sphygmomanometer comprising a sphygmomanometer main body (1) and a sleeve (2) that are detachably coupled to each other, wherein the sphygmomanometer main body (1) is provided with a master controller and a pressure sensor (7) indirectly connected to the sleeve (2),

   wherein the master controller comprises a Bluetooth chip (3),
   wherein the Bluetooth chip (3) integrates:

   a Bluetooth communication module, for having two-way data connection with a mobile terminal, and sending instructions that come from the mobile terminal and are related to blood-pressure measurement to a Bluetooth control module; and
   a Bluetooth control module, for executing control tasks and data processing tasks related to blood-pressure measurement in response to the received instructions related to blood-pressure measurement; wherein, the electronic sphygmomanometer is configured to be able to execute a blood-pressure measurement comprising a first and second measurement stage whereby:

   in a first measurement stage, the Bluetooth communication module is configured to regularly send dynamic air pressure values currently in the sleeve (2) collected by the pressure sensor (7) to the mobile terminal; and

in a second measurement stage, the Bluetooth communication module is configured to associate a blood pressure value, calculated by the Bluetooth control module, with a heart rate value to form a Bluetooth data set and to send the Bluetooth data set to the mobile terminal.

2. The electronic sphygmomanometer of claim 1, further comprising a driving unit, which at least comprises an inflation driver and the integrated air pump (5), wherein

the inflation driver is ocnfigured to drive and/or switch and/or stop working modes of the integrated air pump (5) based on a measurement command issued by the Bluetooth control module, and
the integrated air pump (5) is configured to work alternatively with different working modes based on variation of current polarity of a driving circuit (4) connected thereto.

3. The electronic sphygmomanometer of claim 1 or 2, wherein the integrated air pump (5) has a reversible airflow channel, and the working modes of the integrated air pump (5) include a first working mode in which the sleeve (2) is inflated for pressurization by a motor driven to perform forward rotation and a second working mode in which the sleeve (2) is deflated for depressurization by the motor driven to perform reverse rotation.

4. The electronic sphygmomanometer of any one of claims 1 to 3, wherein the first measurement stage exists when the Bluetooth control module is not yet able to figure out the blood pressure value according to a sequence of the dynamic air pressure values collected by the pressure sensor (7).

5. The electronic sphygmomanometer of any one of claims 1 to 4, wherein while the Bluetooth communication module regularly sends the dynamic air pressure values to the mobile terminal in the second stage, the integrated air pump (5) continuously inflates the sleeve (2) for pressurization.

6. The electronic sphygmomanometer of any one of claims 1 to 5, further comprising a charging unit, which at least comprises a data interface (103) that is configured to have mechanical and/or signal connection with other health measuring equipment (9) so as to provide enriched health monitoring functions.


**Patentansprüche**

1. Ein elektronisches Sphygmomanometer mit integrierter Luftpumpe, welches einen Sphygmomanometer-Hauptkörper (1) und eine Manschette (2) umfasst, die lösbar miteinander verbunden sind, wobei der Sphygmomanometer-Hauptkörper (1) mit einem Master-Controller und einem Drucksensor (7) ausgestattet ist, der indirekt mit der Manschette (2) verbunden ist, wobei der Master-Controller einen Bluetooth-Chip (3) umfasst, der folgendes integriert:

ein Bluetooth-Kommunikationsmodul zur Zwei-Wege-Datenverbindung mit einem mobilen Endgerät und zum Senden von Instruktionen an ein Bluetooth-Steuermodul, wobei die Instruktionen von dem mobilen Endgerät kommen und sich auf die Blutdruckmessung beziehen; und
ein Bluetooth-Steuermodul zum Ausführen von Steuerungsaufgaben und Datenverarbeitungsaufgaben in Bezug auf die Blutdruckmessung als Antwort auf die empfangenen Anweisungen in Bezug auf die Blutdruckmessung,
wobei das elektronische Sphygmomanometer so konfiguriert ist, dass es eine Blutdruckmessung durchführen kann, die eine erste und eine zweite Messstufe umfasst,
wobei das Bluetooth-Kommunikationsmodul in einer ersten Messstufe so konfiguriert ist, dass es regelmäßig dynamische Luftdruckwerte, die aktuell in der Manschette (2) herrschen und vom Drucksensor (7) erfasst werden, an das mobile Endgerät sendet, und
wobei das Bluetooth-Kommunikationsmodul in einer zweiten Messstufe so konfiguriert ist, dass es einen vom Bluetooth-Steuermodul berechneten Blutdruckwert mit einem Herzfrequenzwert verknüpft und so einen Bluetooth-Datensatz bildet und diesen zu dem mobilen Endgerät sendet.

2. Das elektronische Sphygmomanometer nach Anspruch 1, ferner umfassend eine Treibereinheit, die mindestens einen Aufblasantrieb und die integrierte Luftpumpe (5) umfasst,

wobei der Aufblasantrieb so konfiguriert ist, dass er, basierend auf einem von dem Bluetooth-Steuermodul ausgegebenen Messbefehl, die integrierte Luftpumpe (5) antreibt und/oder umschaltet und/oder deren Arbeitsmodi stoppt; und

wobei die integrierte Luftpumpe (5) so konfiguriert ist, dass sie alternativ in verschiedenen Arbeitsmodi arbeitet, basierend auf einer Änderung der Strompolarität einer damit verbundenen Treiberschaltung (4).

3. Das elektronische Sphygmomanometer nach Anspruch 1 oder 2, wobei die integrierte Luftpumpe (5) einen umkehrbaren Luftstromkanal aufweist und die Arbeitsmodi der integrierten Luftpumpe (5) einen ersten Arbeitsmodus umfassen, in dem die Manschette (2) zur Druckbeaufschlagung durch einen zu einer Vorwärtsrotation angetriebenen Motor aufgeblasen wird; und einen zweiten Arbeitsmodus, in dem die Manschette (2) zur Druckentlastung durch den zu einer Rückwärtsrotation angetriebenen Motor entleert wird.

4. Das elektronische Sphygmomanometer nach einem der Ansprüche 1 bis 3, wobei die erste Messstufe vorliegt, wenn das Bluetooth-Steuermodul noch nicht in der Lage ist, den Blutdruckwert entsprechend einer Sequenz der vom Drucksensor (7) erfassten dynamischen Luftdruckwerte zu ermitteln.

5. Das elektronische Sphygmomanometer nach einem der Ansprüche 1 bis 4, wobei das Bluetooth-Kommunikationsmodul in der zweiten Stufe regelmäßig die dynamischen Luftdruckwerte an das mobile Endgerät sendet, während die integrierte Luftpumpe (5) die Manschette (2) zur Druckbeaufschlagung kontinuierlich aufbläst.

6. Das elektronische Sphygmomanometer nach einem der Ansprüche 1 bis 5, ferner umfassend eine Ladeeinheit, die mindestens eine Datenschnittstelle (103) umfasst, die so konfiguriert ist, dass sie mechanisch und/oder signaltechnisch mit anderen Gesundheitsmessgeräten (9) verbunden werden kann, um so erweiterte Gesundheitsüberwachungsfunktionen bereitzustellen.

**Revendications**

1. Sphygmomanomètre électronique comportant pompe à air intégrée, le sphygmomanomètre électronique comprenant un corps principal (1) de sphygmomanomètre et un manchon (2) qui sont accouplés de façon détachable l'un à l'autre, dans lequel le corps principal (1) de sphygmomanomètre est pourvu d'un dispositif de commande principal et d'un capteur de pression (7) indirectement relié au manchon (2),

dans lequel le dispositif de commande principal comprend une puce Bluetooth (3),
dans lequel la puce Bluetooth (3) intègre :

un module de communication Bluetooth, destiné à une connexion de données bidirectionnelle avec un terminal mobile et à envoyer des instructions provenant du terminal mobile et se rapportant à une mesure de tension artérielle à un module de commande Bluetooth ; et
un module de commande Bluetooth, destiné à exécuter des tâches de commande et des tâches de traitement de données se rapportant à une mesure de tension artérielle en réponse aux instructions reçues se rapportant à une mesure de tension artérielle ;
dans lequel le sphygmomanomètre électronique est conçu pour pouvoir exécuter une mesure de tension artérielle comprenant une première et une seconde étape de mesure, moyennant quoi :

dans une première étape de mesure, le module de communication Bluetooth est configuré pour envoyer régulièrement, au terminal mobile, des valeurs de pression d'air dynamique actuellement dans le manchon (2) collectées par le capteur de pression (7) ; et
dans une seconde étape de mesure, le module de communication Bluetooth est configuré pour associer une valeur de tension artérielle, calculée par le module de commande Bluetooth, à une valeur de fréquence cardiaque afin de former un ensemble de données Bluetooth et d'envoyer l'ensemble de données Bluetooth au terminal mobile.

2. Sphygmomanomètre électronique selon la revendication 1, comprenant en outre une unité d'entraînement, qui comprend au moins un dispositif de gonflage et la pompe à air intégrée (5), dans lequel

le dispositif de gonflage est configuré pour entraîner et/ou commuter et/ou arrêter des modes de fonctionnement de la pompe à air intégrée (5) sur la base d'une commande de mesure émise par le module de commande Bluetooth et
la pompe à air intégrée (5) est configurée pour fonctionner alternativement dans différents modes de fonctionnement en fonction d'une variation de polarité de courant d'un circuit d'entraînement (4) qui y est connecté.

3. Sphygmomanomètre électronique selon la revendication 1 ou 2, dans lequel la pompe à air intégrée (5) présente un canal d'écoulement d'air réversible et les modes de fonctionnement de la pompe à air intégrée (5) comportent un premier mode de fonctionnement, dans lequel le manchon (2) est gonflé pour une mise en pression par un moteur entraîné afin d'effectuer une rotation vers l'avant, et un second mode de fonctionnement, dans lequel le manchon (2) est dégonflé pour une décompression par le moteur entraîné afin d'effectuer une rotation inverse.

4. Sphygmomanomètre électronique selon l'une quelconque des revendications 1 à 3, dans lequel la première étape de mesure existe lorsque le module de commande Bluetooth n'est pas encore en mesure de déterminer la valeur de tension artérielle selon une séquence des valeurs de pression d'air dynamique collectées par le capteur de pression (7).

5. Sphygmomanomètre électronique selon l'une quelconque des revendications 1 à 4, dans lequel, tandis que le module de communication Bluetooth envoie régulièrement les valeurs de pression d'air dynamique au terminal mobile dans la seconde étape, la pompe à air intégrée (5) gonfle en continu le manchon (2) pour une mise sous pression.

6. Sphygmomanomètre électronique selon l'une quelconque des revendications 1 à 5 comprenant en outre une unité de charge, qui comprend au moins une interface de données (103) configurée pour présenter une liaison mécanique et/ou une connexion de signal avec d'autres équipements de mesure de santé (9) de manière à fournir des fonctions de surveillance de santé enrichies.

102 —————————————————————— 103

1

2

Fig.1

**EP 4 400 037 B1**

**Fig.2**

19

Fig.3

Fig.4

EP 4 400 037 B1

Fig.5a

Fig.5b

**REFERENCES CITED IN THE DESCRIPTION**

**Patent documents cited in the description**

- CN 111012323 A
- US 20060111639 A1
- CN 209611120 U
- CN 106983503 A
- CN 217682211 U